(19) Europäisches Patentamt
European Patent Office
Office européen des brevets

(11) EP 0 512 416 B1

(12) EUROPÄISCHE PATENTSCHRIFT

(45) Veröffentlichungstag und Bekanntmachung des Hinweises auf die Patenterteilung:
**08.01.1997 Patentblatt 1997/02**

(51) Int Cl.6: **C07F 9/572**

(21) Anmeldenummer: **92107396.1**

(22) Anmeldetag: **30.04.1992**

# (54) Neue homochirale Diphosphine

Homochiral diphosphines

Diphosphines homochiraux

(84) Benannte Vertragsstaaten:
**AT BE CH DE DK ES FR GB GR IT LI LU NL PT SE**

(30) Priorität: **03.05.1991 DE 4114403**

(43) Veröffentlichungstag der Anmeldung:
**11.11.1992 Patentblatt 1992/46**

(73) Patentinhaber: **HOECHST AKTIENGESELLSCHAFT**
**65926 Frankfurt am Main (DE)**

(72) Erfinder:
- **Lerch, Ulrich, Dr.**
  **W-6238 Hofheim am Taunus (DE)**
- **Jendralla, Joachim-Heiner, Dr.**
  **W-6230 Frankfurt am Main 80 (DE)**
- **Seuring, Bernhard, Dr.**
  **W-6238 Hofheim am Taunus (DE)**
- **Henning, Rainer, Dr.**
  **W-6234 Hattersheim am Main (DE)**

(56) Entgegenhaltungen:
**EP-A- 512 415**
**EP-A- 0 151 282**
**EP-A- 0 251 164**

- **CHEMICAL ABSTRACTS, vol. 95, 1981, Columbus, Ohio, US; abstract no. 150889x,**
- **TETRAHEDRON LETTERS Bd. 21, 1980, Seiten 1051 - 1054 I. OJIMA ET AL**
- **H. Christen "Grundlagen der Organischen Chemie (1982), Seiten 229 - 230**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 512 416 B1

## Beschreibung

Homochirale Phosphine werden als Liganden von Übergangsmetallen in einer Reihe von enantioselektiven, katalytischen, organischen Transformationen eingesetzt, z.B. in Hydrierungen, Hydrosilylierungen, Hydroformylierungen, Hydrocyanierungen, Alkylierungen, Aldol-Kondensationen, Cycloadditionen, En-Reaktion und Isomerisierungen [siehe z.B. R. Noyori et al., Acc. Chem. Res. 23, 345 (1990); J. Ojima et al., Tetrahedron 45, 6901 (1989); S.L. Blystone, Chem. Rev. 89, 1663 (1989); H. Brunner, Top Stereochem 18, 129 (1988); H. Brunner, Synthesis, 645 (1988)]. Ferner ist bekannt, daß $C_2$-symmetrische, bidentate, homochirale Diphosphine häufig bessere optische Induktionen als monodentate oder unsymmetrische homochirale Diphosphine bewirken [siehe J. K. Whitesell, Chem. Rev 89, 1581 (1989)].

Die größte Bedeutung besitzen homochirale Diphosphine als Liganden von Ruthenium- oder Rhodium-Ionen in katalytischen, asymmetrischen Hydrierungen [R. Noyori et al., s.o.; H. Brunner, s.o.; K. E. Koenig in "Asymmetric Synthesis", Vol. 5, Ed. Morrison, J.D., Academic Press, Orlando 1985, Seite 71 ff.].

Homochirale Diphosphine mit einer Phenyl-Harnstoff-Gruppierung sind bisher nur in Form des (2S,4S)-Phenyl-CAPP (Formel A) bekannt [J. Ojima et al., Tetrahedron Lett. 21, 1051 (1980) und Tetrahedron 40, 1255 (1984)].

Über Besonderheiten bei der Verwendung von (2S,4S)-Phenyl-CAPP zur asymmetrischen Hydrierung im Vergleich zu (2S,4S)(-)-BPPM wurde bisher nicht berichtet.

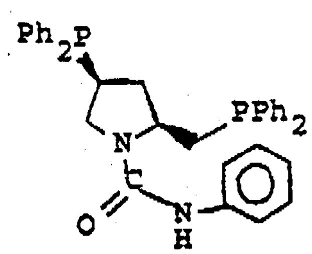

(2S,4S)-(-)-Phenyl-CAPP

(Formel A)

Gegenstand der vorliegenden Anmeldung ist eine Verbindung der Formel (Z),

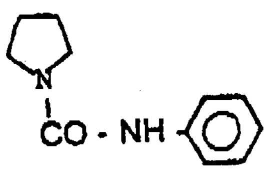

(Z)

welche in Pyrrolidinteil zwei Biarylphosphor-Substituenten W enthält,

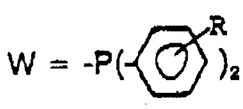

wobei R für Wasserstoff oder ($C_1$-$C_5$)Alkyl steht.

Diese Verbindung hat eine Struktur aus einer der folgenden Gruppen I oder II:

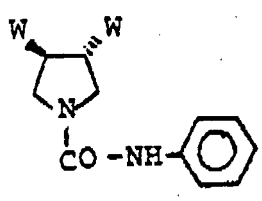

(I)

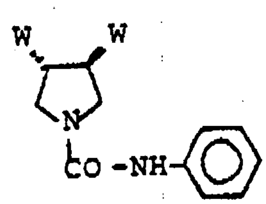

(II)

Insbesondere betrifft die vorliegende Erfindung folgende neuen homochiralen Diphosphine der Formel 1 - 2, die eine Phenyl-Harnstoff-Gruppierung als funktionelle Gruppe enthalten:

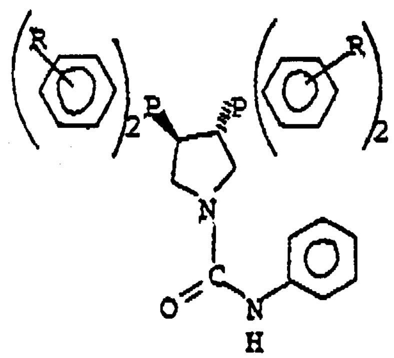

1

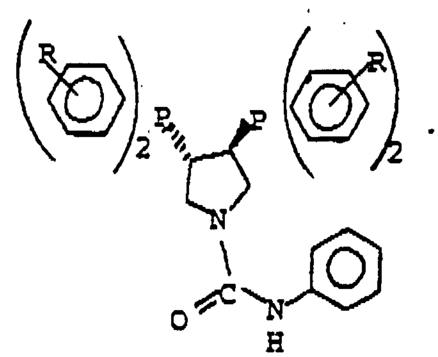

2

Die beiden neuen $C_2$-symmetrischen Verbindungen 1 und 2 stehen im Verhältnis von Bild und Spiegelbild.

In den Formeln 1 - 2 bedeutet R Wasserstoff oder ($C_1$-$C_5$)-Alkyl, insbesondere jedoch Wasserstoff oder Methyl.

Die Verbindungen 1 - 2 können aus bekannten, in 1-Position unsubstituierten Bis-(diarylphosphino)-pyrrolidinen durch Umsetzung mit Phenylisocyanat in praktisch quantitativer Ausbeute erhalten werden. (3R,4R)-(+)-3,4-Bis-(diphenylphosphino)-pyrrolidiniumchlorid (Formel 3, R = H) wird gemäß U. Nagel et al. (Chem Ber. 119, 3326 (1986)) aus käuflicher (+)-Weinsäure hergestellt. Die Verbindung der Formel 3 mit R = Methyl wird analog bei Verwendung von Ditolylphosphan erhalten. Das Enantiomer (Formel 4) wird entsprechend aus käuflicher (-)-Weinsäure hergestellt.

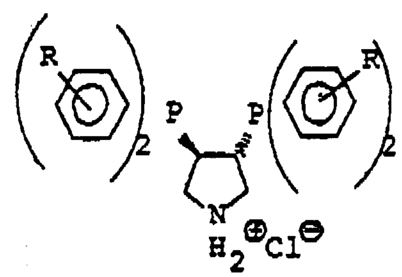

3

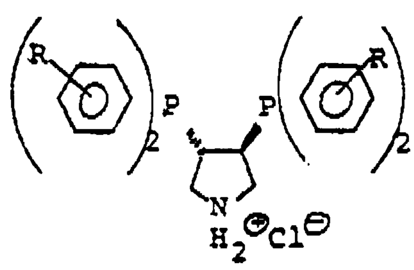

4

Die Verbindungen 3 und 4 sowie einige Analoga und deren Verwendung zur asymmetrischen Hydrierung werden in DE 34 03 194 und DE 34 46 303 beschrieben. Verbindungen mit Harnstoff-Funktionalität, z.B. 1 oder 2, wie sie der vorliegenden Erfindung zugrundeliegen, werden im Stand der Technik nicht beschrieben.

Die zeitgleich von der gleichen Anmelderin eingereichte Patentanmeldung "Verfahren zur enantioselektiven Synthese von 2(R)-Benzylbernsteinsäuremonoamid-Derivaten" (DE-A .. .. ...) beschreibt die Verwendung der homochiralen Diphosphine 1 - 2 in Form ihrer neutralen oder kationischen Rhodium(I)-komplexe zur asymmetrischen Hydrierung von Phenylitaconsäuren der Formel 5 und deren Monoamiden der Formel 6.

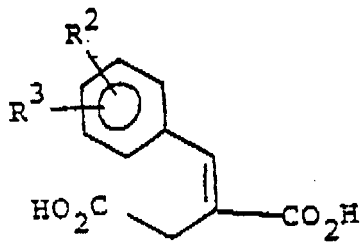

5

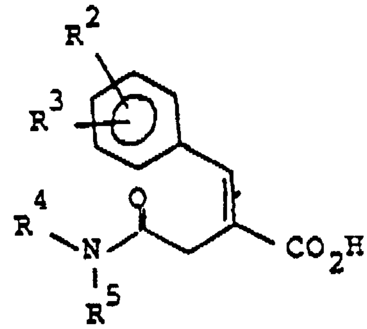

6

Auf diese Patentanmeldung wird ausdrücklich Bezug genommen.
Die Verwendung der Phosphine 1 - 2 zur asymmetrischen Hydrierung hat große Vorteile gegenüber dem Einsatz von konventionellen homochiralen Diphosphinen. Ein besonderer Vorteil von 1 - 2 ist, daß mit diesen Diphosphinen sowohl die Monoamide 6, wie auch die Dicarbonsäuren 5, enantioselektiv hydriert werden können während mit konventionellen Diphosphinen wie BPPM, DIOP, Phenyl-β-glup an den Monoamiden 6 nur geringe asymmetrische Induktionen erzielt werden können.

Die der Erfindung zugrundeliegenden Diphosphine sind als chirale Hilfsstoffe bei der Herstellung von optisch aktiven Verbindungen besonders geeignet, da sie zu hohen Enantiomerenüberschüssen (ee) führen, was beispielsweise bei der Synthese von Wirkstoffen, wie z.B. pharmazeutischen Präparaten, oder deren Vorstufen von großer Bedeutung ist.

Beispiele

Herstellung der chiralen Diphosphine, sowie neuer Rhodium-Katalysatoren

1. Herstellung von (3R,4R)(+)-1-Phenylaminocarbonyl-3,4-bis-(di-p-tolyl phosphino)-pyrrolidin (Formel 1, R = $CH_3$)

a1) (3S,4S)-3,4-Bis(methylsulfonyloxy)-pyrrolidinium-bromid wird gemäß DE-A 34 03 194 aus L-(+)-Weinsäure hergestellt.

a2) (3R,4R)-3,4-Bis-(di-p-tolylphosphino)-pyrrolidinium-chlorid

Zur Lösung von 33,15 g (155 mmol) Di-(p-tolyl)-phosphan in 150 ml abs. THF werden 3,56 g (155 mmol) Natrium gegeben und 5 Stunden unter $N_2$ unter Rückfluß gekocht, wobei das Natrium weitgehend unter Reaktion in Lösung geht. Das verbliebene Natrium (0,2 g) wird entnommen und das THF im Vakuum entfernt. Der Rückstand wird in 160 ml abs., entgastem, auf 0°C vorgekühltem DMF gelöst, auf -30°C abgekühlt und auf einmal mit 13,6 g (40 mmol) des Hydrobromids aus a1) versetzt. Der Ansatz wird über Nacht bei 0°C unter Feuchtigkeitsausschluß im Kühlschrank aufbewahrt. Das DMF wird im Ölpumpenvakuum bei 40°C Badtemperatur abgezogen. Der feste, rötliche, zähe Rückstand wird mit 140 ml Wasser und 140 ml Ether (beide entgast) versetzt, die organische Phase abgetrennt, die Wasserphase noch einmal mit 70 ml Ether extrahiert und die vereinigten Etherphasen mit 160 ml 1N Salzsäure über Nacht unter Argon gerührt. Der weiße Feststoff wird abgesaugt, im HV getrocknet und dann aus 170 ml Isopropanol (entgast) umkristallisiert. Die Kristalle werden abgesaugt, mit Isopropanol nachgewaschen und im HV getrocknet.
Ausbeute: 12,7 g (60 % d. Th.). Bei ca. 200 Torr im Rohr eingeschmolzene Kristalle zeigen einen Schmp. von 238-243°C, beim Aufheizen im geöffneten Schmelzpunktrohr wird Schmp. 224 - 228°C gefunden.
$[\alpha]_D^{20}$ = +171,6° (c = 2,67 in 99 %igem Ethanol).
$^1$H-NMR ($CDCl_3$): δ = 10.02 (br. s, 2H, $NH_2^+$), 7.30 (m, 4H, arom.-H), 6.93-7.18 (m, 8H, arom.-H), 3.58 (m, 2H, $C\underline{H}_2NH$), 3.12 (m, 2H, $C\underline{H}_2NH$), 2.88 (m, 2H, $C\underline{H}PAr_2$), 2.36 (s, 6H, $C\underline{H}_3$), 2.31 (s, 6H, $CH_3$) ppm.

a3) (3R,4R)(+)-3,4-Bis(di-p-tolyl-phosphino)-pyrrolidin
Zum entgasten 2-Phasengemisch von 110 ml Diethylether und 27 ml (5.4 mmol) 0,2N Kalilauge werden 2,02 g (3.80 mmol) des Hydrochlorids aus a2) gegeben und unter Argon gerührt bis vollständige Lösung eintritt (ca. 10 Min.). Die Etherphase wird abgetrennt, die wäßrige Phase mit 50 ml entgastem Diethylether extrahiert.

Die vereinigten Etherphasen werden getrocknet, das Lösungsmittel im Vakuum entfernt und der Rückstand im HV getrocknet,. Man erhält 1,95 g (3.9 mmol, Ausbeute 100 %) weißen Schaum, Schmp. 63-65°C.
$^1$H-NMR ($CDCl_3$): δ = 7.28 (t, 4H, arom.-H), 7.14 (d, 4H, arom.-H), 6.90-7.16 (m, 8H, arom.-H), 4.83 (s, 1H, NH), 3.23-3.40 (m, 1H, $Ar_{32}PC\underline{H}$), 2.70-2.88 (m, 4H, $NC\underline{H}_2$), 2.28-2.42 (m, 1H, $Ar_2PC\underline{H}$), 2.30 (s, 12H, $CH_3$) ppm.
Die Verbindung wurde ohne weitere Reinigung umgesetzt.

a4) (3R,4R)(+)-1-Phenylaminocarbonyl-3,4-bis(di-p-tolyl-phosphino)-pyrrolidin

Zur Argon-durchperlten Lösung von 1,87 g (4.0 mmol) des Produktes aus a3) in 30 ml Dichlormethan tropft man unter Eiskühlung 500 mg (4.2 mmol) Phenylisocyanat und rührt dann 30 Minuten bei Raumtemperatur. Man tropft 10 ml Wasser zu, rührt 15 Minuten, trennt die wäßrige Phase ab und wäscht die organische Phase nach Verdünnen mit 60 ml entgastem Dichlormethan mit zweimal je 20 ml 2N Salzsäure, dann mit 20 ml Wasser. Man trocknet die organische Phase, entfernt das Lösungsmittel im Vakuum und trocknet den Rückstand im Hochvakuum. Man erhält 2.08 g (3.55 mmol), 89 % d.Th.) Rohprodukt. Dieses wird in 5 ml Dichlormethan gelöst und 40 ml entgastes n-Hexan zugetropft, wobei eine bleibende Trübung eintritt. Die Suspension wird 12 Stunden auf 0°C gekühlt, der Feststoff abgesaugt, mit n-Hexan gewaschen und im HV getrocknet. Man erhält 1,92 g (3.12 mmol, 83 % d.Th.) farblosen Feststoff, $[\alpha]_D^{20}$ = 93,2° (c = 2.04, Benzol). Beim Erwärmen erfolgt Zersetzung unter Gasentwicklung bei 110-120°C. DC:
Cyclohexan/Ethylacetat 3:1, $R_f$:0.40.
$^1$H-NMR (DMSO-$d_6$): δ = 8.14 (s, 1H, NH), 6.85-7.48 (m, 21H, arom.-H), 3.84 (m, 2H, $NC\underline{H}_2$), 3.38 (t, 2H, $NC\underline{H}_2$), 2.85 (t, 2H, $C\underline{H}PAr_2$), 2.30 (s, 6H, 2 x $CH_3$), 2.28 (s, 6H, 2 x $CH_3$) ppm.

a5) [(3R,4R)(+)-1-Phenylaminocarbonyl-3,4-bis-(di-p-tolylphosphino)-pyrrolidin-P,P'](1,5-cyclooctadien)rhodium-tetrafluoroborat

Zur Lösung von 630 g (1.55 mmol) Bis-(1,5-cyclooctadien)-rhodium-tetrafluoroborat in 6 ml entgastem Dichlormethan gibt man unter Argon bei Raumtemperatur die Lösung von 935 mg (1.52 mmol) des Produktes aus a4) in 23 ml Dichlormethan. Man rührt 2 Stunden. DC-Kontrolle zeigt quantitative Umsetzung des Phosphins zum Rhodium-Komplex an. Das Lösungsmittel wird im Vakuum entfernt, der Rückstand in 6 ml Methanol aufgenommen und der Suspension 150 ml Ether zugesetzt. Man rührt 15 Minuten in Argon, saugt den Feststoff ab, wäscht ihn mit Ether und trocknet ihn im HV. Man erhält 1,35 g (1.48 mmol), 97 % d.Th.) orangegelbes Pulver.
$^{31}$P-NMR ($CD_2Cl_2$): gegen 85 %iges $H_3PO_4$ als externer Standard
δ = 32.8 ppm (d, $J_{Rh,P}$ = 150.3 Hz)

2. Herstellung von (3R,4R)(+)-1-Phenylaminocarbonyl-3,4-bis-(diphenylphosphino)-pyrrolidin (Formel 1, R = H)

b1) (3R,4R)(+)-3,4-Bis-(diphenylphosphino)-pyrrolidiniumchlorid
wird in Analogie zu a2) hergestellt.
Ausbeute 74 % d.Th., Schmp. 207-209°C, $[\alpha]_D^{25}$ = + 163° (c = 3.0 in 99 %igem Ethanol).
$^1$H-NMR ($CDCl_3$): δ = 9-11 (s, sehr breit, 2H, $\underline{N}H_2^+$), 7.1-7.6 (m, 20H, arom.-H), 3.62 (m, 2H, $NC\underline{H}_2$), 3.16 (m, 2H, $NC\underline{H}_2$), 2.95 (m, 2H, $C\underline{H}PPh_2$).

b2) (3R,4R)(+)-3,4-Bis-(diphenylphosphino)-pyrrolidin
wird aus b1) in Analogie zu a3) hergestellt.
Das Rohprodukt hat Schmp. 120-122°C.
$^1$H-NMR ($CD_3OD$): δ = 7.1-7.5 (m, 20H, arom.-H), 4.82 (s, 1H, NH), 3.30-3.45 (s, 2H, $C\underline{H}PPh_2$), 2.75-2.93 (m, 4H, $CH_2$) ppm.

b3) (3R,4R)(+)-1-Phenylaminocarbonyl-3,4-bis-(diphenylphosphino)-pyrrolidin
wird aus dem Rohprodukt b2) in Analogie zu a4) hergestellt.
Ausbeute: 100 %, Schmp. 165-167°C, DC (Cyclohexan/Ethylacetat 3:2) $R_f$ = 0.48.
$^1$H-NMR (DMSO-$d_6$): δ = 8.20 (s, 1H, NH), 7.05-7.50 (m, 24H, arom.-H), 6.90 (m, 1H, arom.-H), 3.90 (m, 2H, $NC\underline{H}_2$), 3.43 (t, 2H, $NC\underline{H}_2$), 2.92 (t, 2H, $C\underline{H}PPh_2$) ppm.

b4) [(3R,4R)(+)-1-Phenylaminocarbonyl-3,4-bis-(diphenylphosphino)-pyrrolidin-P,P'](1,5-cyclooctadien)-rhodium-tetrafluoroborat
wird aus dem Produkt b3) in Analogie zu a5) hergestellt.

Gelboranges Pulver, Ausbeute: 91 % d.Th..
MS (FAB, 3-NBA): m/e = 769 (Molekülpeak des Kations).
$^1$H-NMR ($CD_2Cl_2$): $\delta$ =7.98 (m, 4H, arom.-H), 7.52-7.80 (m, 12H, arom.-H), 7.45 (m, 4H, arom.-H), 7.31 (dd, 2H, arom.-H), 7.15 (2, 2H, arom.-H), 6.91 (m, angenähert t, 1H, arom.-H), 6.64 (s, 1H, NH), 5.20 (s, breit, 2H, olef.-H), 4.56 (qua, 2H, olef.-H), 3.75 (m, 2H, $NCH_2$), 3.14 (m, 2H, $NCH_2$), 2.99 (m, 2H, $CHPPh_2$), 2.30-2.65 (m, 4H, allyl-H von COD), 2.05-2.30 (m, 4H, allyl-H von COD) ppm.
$^{31}$P-NMR ($CD_2Cl_2$ gegen 85 %ige $H_3PO_4$ als externer Standard):
$\delta$ = 34.1 ppm (d, $J_{Rh,P}$ = 149.0 Hz, Halbwertsbreite ca. 30 Hz)

3. Herstellung von (3S,4S)(-)-Phenylaminocarbonyl-3,4-bis-(diphenylphosphino)-pyrrolidin (Formel 2, R = H)

c1) (3R,4R)-3,4-Bis-(methylsufonyloxy)-pyrrolidinium-bromid wird gemäß DE-A 34 03 194 aus D-(-)-Weinsäure hergestellt.

c2) (3S,4S)(-)-3,4-Bis-(diphenylphosphino)-pyrrolidiniumchlorid
wird in Analogie zu a2) hergestellt.
Ausbeute 78 % d.Th., Schmp. 205-208°C, $[\alpha]_D^{25}$ = -162° (c = 3,0 in 99 %igem Ethanol).

c3) (3S,4S)(-)-3,4-Bis-(diphenylphosphino)-pyrrolidin
wird aus c2) in Analogie zu a3) hergestellt.
Das Rohprodukt hat Schmp. 121-124°C.

c4) (3S,4S)(-)-Phenylaminocarbonyl-3,4-bis-(diphenylphosphino)-pyrrolidin
wird aus dem Rohprodukt c3) in Analogie zu a4) hergestellt.
Ausbeute: 100 %, Schmp. 164-166°C, DC (Cyclohexan/Ethylacetat 3:2) $R_f$= 0.48.
$^1$H-NMR identisch mit c3).

**Patentansprüche**

**1.** Verbindung der Formel Z

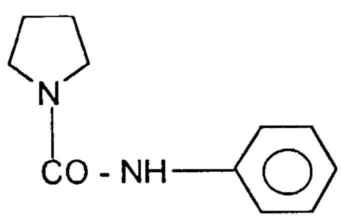

(Z)

welche im Pyrrolidinteil zwei phosphorhaltige Substituenten W enthält,

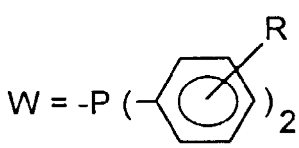

,

wobei R für Wasserstoff oder ($C_1$-$C_6$)-Alkyl steht,
dadurch gekennzeichnet, daß sie aus einer der folgenden Gruppen I oder III ausgewählt wird:

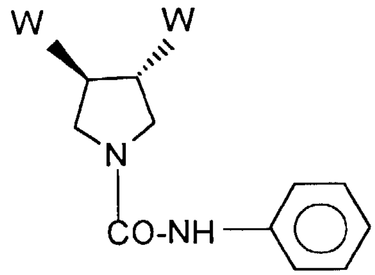

(I)

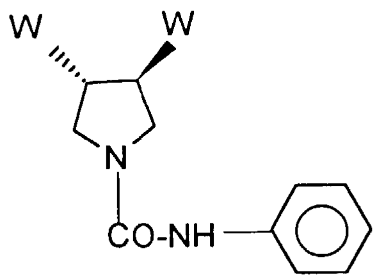

(II)

2. Verbindung gemäß Anspruch 1, dadurch gekennzeichnet, daß die Verbindung der Formel (Z) folgende Struktur (1) aufweist,

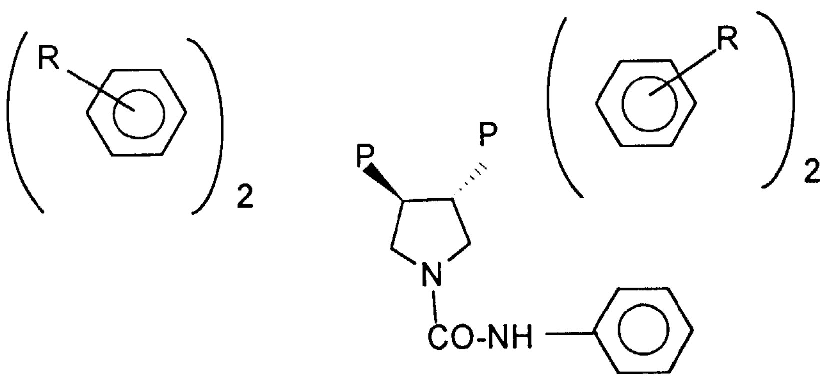

(1)

wobei R gleich Wasserstoff oder Methyl bedeutet.

3. Verbindung gemäß Anspruch 1, dadurch gekennzeichnet, daß die Verbindung der Formel (Z) folgende Struktur (2) aufweist,

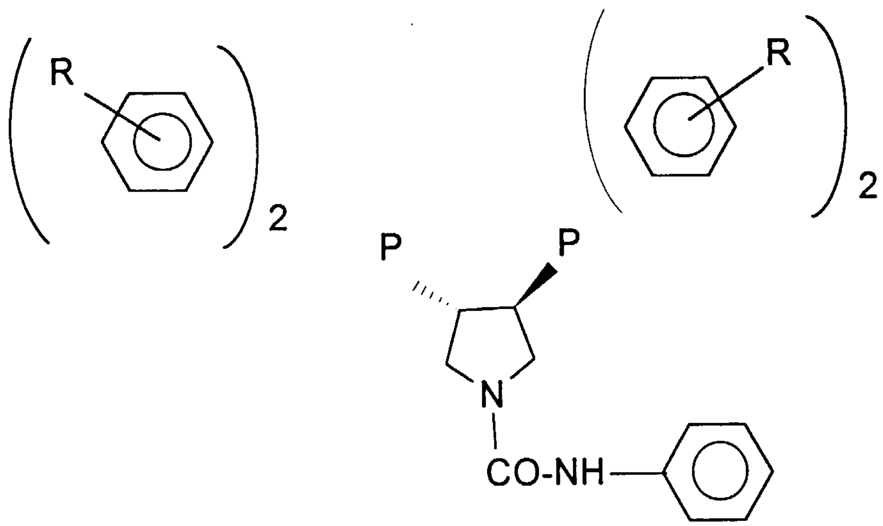

(2)

wobei R gleich Wasserstoff oder Methyl bedeutet.

4. Verfahren zur Herstellung von Verbindungen der Formeln (1) oder (2) gemäß Ansprüchen 2 oder 3 durch Umsetzung von in 1-Position unsubstituierten Diarylphosphinpyrrolidinen mit Phenylisocyanat.

**Claims**

1. A compound of the formula Z

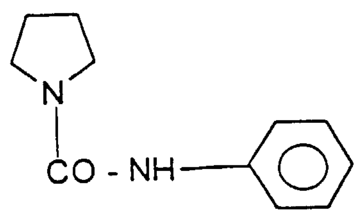

(Z)

which contains two phosphorus-containing substituents W in the pyrrolidine moiety,

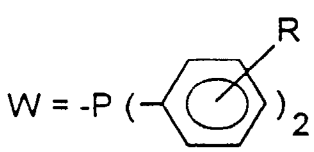

,

in which R is hydrogen or ($C_1$-$C_6$)-alkyl,
which compound is selected from one of the following groups I or II:

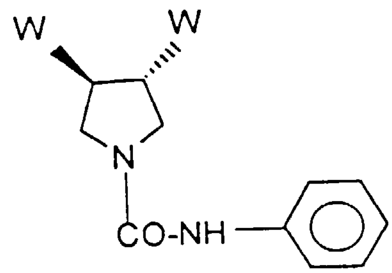

(I)

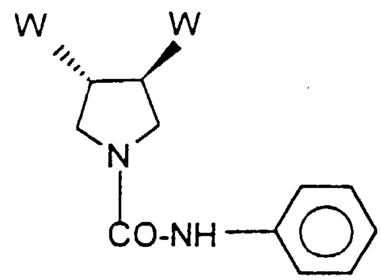

(II)

**2.** A compound as claimed in claim 1, wherein the compound of the formula (Z) has the following structure (1)

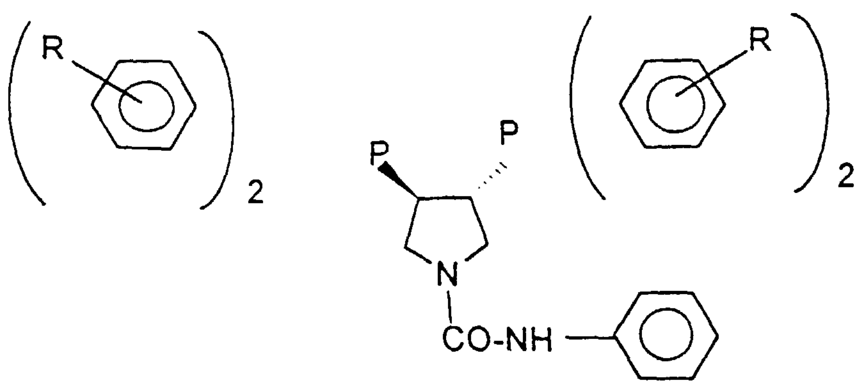

(1)

in which R is hydrogen or methyl.

**3.** A compound as claimed in claim 1, wherein the compound of the formula (2) has the following structure (2)

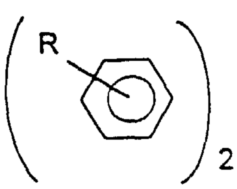

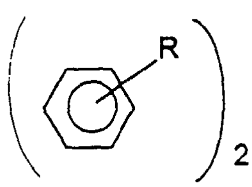

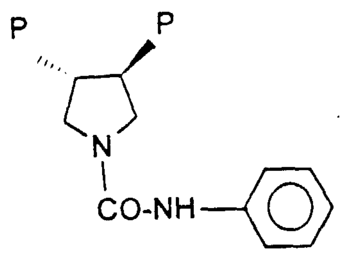

(2)

in which R is hydrogen or methyl.

4. A process for the preparation of a compound of the formula (1) or (2) as claimed in claims 2 or 3 by reaction of diarylphosphinopyrrolidines which are unsubstituted in the 1-position with phenyl isocyanate.

**Revendications**

1. Composé de formule Z

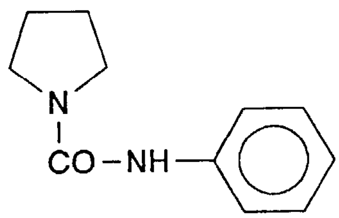

(Z)

qui comporte dans la partie pyrrolidine deux substituants phosphorés W,

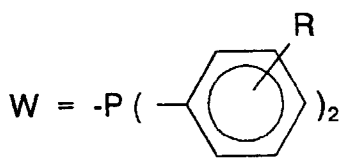

où R représente un atome d'hydrogène ou un groupe alkyle ($C_1$-$C_6$), caractérisé en ce qu'il est choisi dans le groupe I ou II suivant :

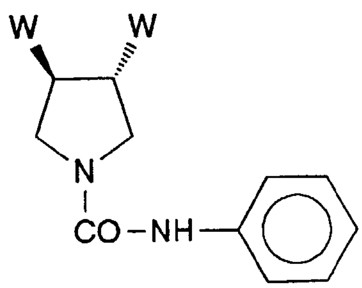

(I)

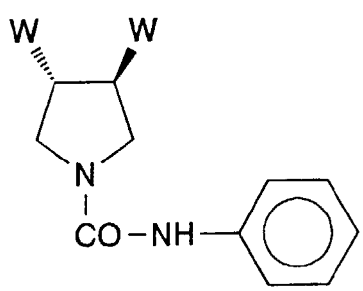

(II)

2. Composé selon la revendication 1, caractérisé en ce que le composé de formule (Z) présente la structure (1) suivante :

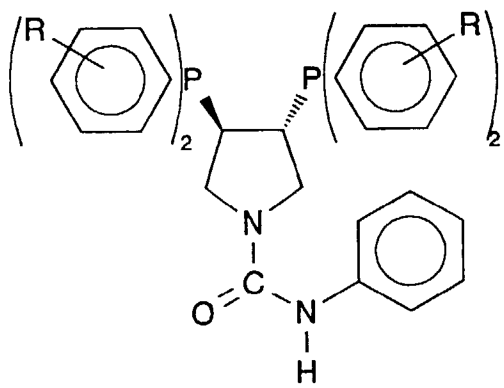

(1)

où R représente un atome d'hydrogène ou un groupe méthyle.

3. Composé selon la revendication 1, caractérisé en ce que le composé de formule (Z) présente la structure (2) suivante :

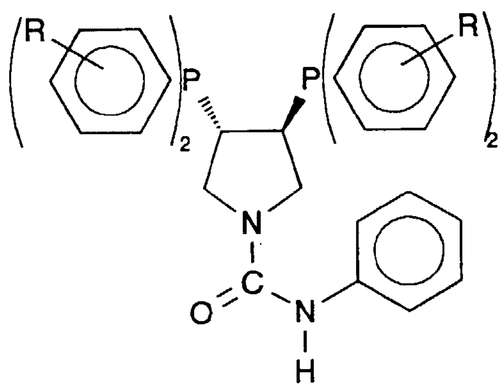

(2)

où R représente un atome d'hydrogène ou un groupe méthyle.

4. Procédé pour la préparation de composés de formule (1) ou (2) selon la revendication 2 ou 3, par réaction de diarylphosphine-pyrrolidines non substituées en position 1 avec du phénylisocyanate.